(19) Europäisches Patentamt
European Patent Office
Office européen des brevets

(11) **EP 2 593 219 B1**

(12) **EUROPÄISCHE PATENTSCHRIFT**

(45) Veröffentlichungstag und Bekanntmachung des Hinweises auf die Patenterteilung:
**30.05.2018 Patentblatt 2018/22**

(21) Anmeldenummer: **11736316.8**

(22) Anmeldetag: **08.07.2011**

(51) Int Cl.:
*B01J 21/04* *(2006.01)*     *B01J 29/40* *(2006.01)*
*B01J 35/02* *(2006.01)*     *B01J 37/00* *(2006.01)*
*C07C 1/20* *(2006.01)*

(86) Internationale Anmeldenummer:
**PCT/EP2011/061679**

(87) Internationale Veröffentlichungsnummer:
**WO 2012/007398 (19.01.2012 Gazette 2012/03)**

(54) **VERFAHREN ZUR HERSTELLUNG VON KATALYSATOREN AUF ZEOLITHBASIS ZUR UMSETZUNG VON OXYGENATEN ZU NIEDEREN OLEFINEN**

PROCESS FOR PRODUCING ZEOLITE-BASED CATALYSTS FOR CONVERSION OF OXYGENATES TO LOWER OLEFINS

PROCÉDÉ DE PRODUCTION DE CATALYSEURS À BASE DE ZÉOLITE POUR TRANSFORMER DES COMPOSÉS OXYGÉNÉS EN OLÉFINES INFÉRIEURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2010 DE 102010026880**

(43) Veröffentlichungstag der Anmeldung:
**22.05.2013 Patentblatt 2013/21**

(73) Patentinhaber: **Süd-Chemie IP GmbH & Co. KG 81925 München (DE)**

(72) Erfinder:
• **BURGFELS, Götz**
  **83043 Bad Aibling (DE)**
• **FRAUENRATH, Manfred**
  **83109 Großkarolinenfeld (DE)**
• **ROTHÄMEL, Martin**
  **60437 Frankfurt am Main (DE)**
• **HAAG, Stéphane**
  **60598 Frankfurt am Main (DE)**
• **POHL, Sven**
  **60388 Frankfurt am Main (DE)**

(74) Vertreter: **Clariant Produkte (Deutschland) GmbH IPM / Patent & License Management Arabellastrasse 4a 81925 München (DE)**

(56) Entgegenhaltungen:
EP-A1- 1 424 128     EP-A1- 1 892 229
WO-A1-2004/030815    WO-A1-2011/061196

EP 2 593 219 B1

**Beschreibung**

Die Erfindung betrifft ein Herstellungsverfahren für Katalysatoren auf Zeolithbasis und die Verwendung der Katalysatoren bei der Umsetzung von Oxygenaten zu niederen Olefinen, insbesondere von Methanol zu Propylen.

Katalysatoren auf der Basis von kristallinen Alumosilicaten, die aus einer Aluminiumquelle, einer Siliciumquelle, einer Alkaliquelle, einem Templat (z.B. einer Tetrapropylammonium-Verbindung) und Wasser hergestellt werden, sind aus der US 3 702 886 bekannt.

Die DE-A-28 22 725 beschreibt die Herstellung von Methanolumwandlungskatalysatoren auf der Basis von kristallinen Alumosilicaten. Der Durchmesser der Primärkristallite liegt bei 1 $\mu$m und mehr. Bezweckt wird die Herstellung von Primärkristalliten mit Durchmessern von weit über 1 $\mu$m. Dazu muss das Kristallwachstum durch höhere Temperaturen gefördert und die Keimbildung durch geringe Konzentrationen der für die Kristallisation der Zeolithe wesentlichen Template gehemmt werden. Es finden sich keine Hinweise auf die Verwendung von Bindemitteln oder über die Größe der Agglomerate.

Nach der DE-A-24 05 909 (FR-A-2 217 408) werden Katalysatoren zur Kohlenwasserstoffumwandlung auf der Basis von Zeolithen von ZSM-5-Typ hergestellt, wobei die mittleren Durchmesser der Primärkristallite im Bereich von 0,005 bis 0,1 $\mu$m liegen.

Aus den Primärkristalliten werden Agglomerate in der Größenordnung von 0,1 bis 1 $\mu$m erzeugt. Zur Herstellung der Katalysatoren werden die Agglomerate u. a. mit Aluminiumoxid als Bindemittel versetzt, wobei aber auch andere Bindemittel als gleichwertig angegeben sind. Über die Teilchengröße der Bindemittel finden sich keine Angaben. Weiterhin wurde die Synthese in Anwesenheit von Schwefelsäure und unter Verwendung von $Al_2(SO_4)_3 \cdot xH_2O$ durchgeführt.

Die US-A-4 025 572 betrifft ein Verfahren zur Herstellung bestimmter Kohlenwasserstoffgemische, wobei das Katalysatorbett u. a. einen Zeolith enthält. Die Synthese des Zeolithen wird in Anwesenheit von Schwefelsäure und unter Verwendung von $Al_2(SO_4)_3 \cdot xH_2O$ durchgeführt. Nach einem Beispiel wird der Zeolithkatalysator mit 90 Gew.-% Aluminiumoxid gemischt und pelletiert.

Die EP 0 369 364 A1 beschreibt Katalysatoren auf Basis von kristallinen Alumosilicaten vom Pentasiltyp mit einem Si/Al-Atomverhältnis von mindestens 10, die aus Primärkristalliten mit einem mittleren Durchmesser von mindestens 0,1 $\mu$m und höchstens 0,9 $\mu$m aufgebaut sind. Die Größe der Primärkristallite wird als wichtig für die Lebensdauer der Katalysatoren angesehen. Als Anwendungsbeispiele werden ein Methanol-zu-Olefin-Verfahren und ein Olefin-zu-Diesel-Verfahren angegeben.

In der EP 1 892 229 A1 wird die Umwandlung eines Kohlenwasserstoffstroms in Propylen offenbart. Der eingesetzte Katalysator umfasst mindestens einen Zeolithen und einen alumina-basierten Träger und liegt in Form sphärischer Kugeln vor. Der Katalysator wird mittels "oil-drop"-Verfahren hergestellt.

Die WO 2011/061196 A1 beschreibt einen Katalysator auf Basis von Alumosilicaten vom Pentasil-Typ in Form von Kugeln mit einem mittleren Durchmesser zwischen 0,3 und 7 mm, einer BET-Oberfläche zwischen 300 und 600 $m^2$/g und enthaltend ein Bindemittel. Katalysatoren auf Basis von Kugeln des genannten Durchmessers werden als weniger anfällig gegenüber Deaktivierungen angesehen.

Die europäische Patentanmeldung EP 1 424 128 A1 offenbart Katalysatoren auf Basis von kristallinem Alumosilicat, die Primärkristallite mit einem mittleren Durchmesser von mindestens 0,01 $\mu$m und weniger als 0,1 $\mu$m und ein Porenvolumen von 0,3 bis 0,8 $cm^3$/g aufweisen. Diese Katalysatoren lassen sich prinzipiell im Methanol-zu-Olefin-Verfahren im Labormaßstab verwenden. Sie haben aber den Nachteil, dass sie eine vergleichsweise geringe mechanische Festigkeit, insbesondere eine Seitendruckfestigkeit von kleiner als 0,8 kp/mm (7,8 N/mm) aufweisen. Dies kann insbesondere zu Problemen beim Transport sowie beim Befüllen und Betrieb von industriellen Reaktoren führen, da dabei die Katalysatoren auseinander brechen können, was eine vergleichsweise geringe Lebensdauer bedingt.

Der Erfindung liegt demgegenüber die Aufgabe zugrunde, ein Verfahren zur Herstellung von Katalysatoren auf der Basis von kristallinen hochaktiven Alumosilicaten bereitzustellen, die bei katalytischen Verfahren, insbesondere bei Methanol-zu-Propylen-(MTP-)Umwandlungsverfahren, ein Optimum an Aktivität und verbesserter Stabilität bzw. Festigkeit und somit sehr guter Lebensdauer zeigen. Diese Aufgabe wird durch das erfindungsgemäße Verfahren und die damit erhältlichen Katalysatoren gelöst.

Die Erfindung betrifft ein Verfahren zur Herstellung eines Katalysators auf der Basis von kristallinem Alumosilicat vom Pentasil-Typ mit einem Si/Al-Atomverhältnis zwischen 10 und 100, umfassend die Schritte:

(a) Behandeln von Aluminiumoxidhydrat mit einem wässrigen säurehaltigen Medium,
(b) Vermischen des mit wässrigem säurehaltigem Medium behandelten Aluminiumoxidhydrats aus Schritt (a) mit einem H-Zeolithen mit einem mittleren Durchmesser der Primärkristallite von zwischen 0,01 $\mu$m und weniger als 0,1 $\mu$m und
(c) Kalzinieren der in Schritt (b) erhaltenen Mischung, wobei mindestens 95 Vol.-% der Teilchen des Aluminiumoxidhydrats (auf den mittleren Durchmesser bezogen) kleiner oder gleich 100 $\mu$m ist.

Dabei wurde überraschend gefunden, dass das erfindungsgemäße Verfahren in Katalysatoren mit gesteigerter Lebensdauer durch erhöhte Seitendruckfestigkeit, sowie hervorragender Selektivität und Aktivität resultiert. Durch das Vorvermischen des Aluminiumoxidhydrats mit dem wässrigen säurehaltigen Medium in Schritt (a) des erfindungsgemäßen Verfahrens wird eine signifikante Verbesserung der katalytischen Eigenschaften der erfindungemäßen Katalysatoren erzielt, verglichen mit Katalysatoren, die durch Zugabe von wässrigem säurehaltigem Medium zu einer Mischung aus H-Zeolith und Aluminiumoxidhydrat erhältlich sind.

Das in Schritt (a) eingesetzte Aluminiumoxidhydrat ist zum Beispiel durch Hydrolyse von aluminiumorganischen Verbindungen, wie z.B. Aluminiumtrialkylen oder Aluminiumalkoholaten, erhältlich und ist vorzugsweise Na- und Fe-arm. Eingesetzt wird feinteiliges Aluminiumoxidhydrat mit einem Korngrößenspektrum von mindestens 95% Vol.-% ≤ 100 $\mu$m. Diese Werte sind, gemittelt über eine Vielzahl von Kristalliten, jeweils auf den mittleren Durchmesser bezogen, der wie der mittlere Durchmesser der Primärkristallite nachstehend definiert ist. Im Einzelnen hat das Aluminiumoxidhydrat typischerweise folgendes Korngrößenspektrum:

91 Vol.-% ≤ 90 $\mu$m
51 Vol.-% ≤ 45 $\mu$m
27 Vol.-% ≤ 25 $\mu$m.

Vorzugsweise sind mindestens 97 Vol.-% der Teilchen des Aluminiumoxidhydrats (auf den mittleren Durchmesser bezogen) kleiner oder gleich 100 $\mu$m.

Als wässriges säurehaltiges Medium dienen prinzipiell wässrige organische oder anorganische Säuren mit einem Konzentrationsbereich von 0,1% bis 100%, welche nötigenfalls mit Wasser verdünnt werden. Beispielsweise können organische Säuren wie Essigsäure oder anorganische Säuren wie Salpetersäure etc. zur Bildung des wässrigen säurehaltigen Mediums eingesetzt werden.

Das Mengenverhältnis zwischen Aluminiumoxidhydrat und wässrigem säurehaltigem Medium unterliegt keiner speziellen Beschränkung. Vorzugsweise wird jedoch das wässrige säurehaltige Medium zu dem Aluminiumoxidhydrat in einer derartigen Menge zugegeben, dass sich eine Säurekonzentration von 0,005 bis 2,5 mol H$^+$/mol Al$_2$O$_3$, vorzugsweise 0,01 bis 1,5 mol H$^+$/mol Al$_2$O$_3$ und insbesondere 0,05 bis 1,0 mol H$^+$/mol Al$_2$O$_3$ einstellt.

Der bei dem erfindungsgemäßen Verfahren in Schritt (b) eingesetzte H-Zeolith ist durch ein Verfahren erhältlich, das die folgenden Schritte umfasst:

(i) Herstellen von Alumosilicat-Kristalliten mit einem mittleren Durchmesser von größer oder gleich 0,01 $\mu$m bis weniger als 0,1 $\mu$m durch Umsetzen einer Siliziumquelle, einer Aluminiumquelle, einer Alkaliquelle und einem Templat bei einer Reaktionstemperatur von größer oder gleich 90°C,
(ii) Abtrennen und gegebenenfalls Zwischenkalzinieren der Kristallite aus Schritt (i),
(iii) Umsetzen der Alumosilicat-Kristallite aus Schritt (ii) mit einer Ionenaustauschverbindung,
(iv) Abtrennen und gegebenenfalls Kalzinieren des aus Schritt (iii) erhaltenen Produkts.

Als Siliziumquelle, Aluminiumquelle, Alkaliquelle und Templat können jeweils beliebige der im Stand der Technik üblicherweise zur Herstellung von Alumosilicaten verwendeten Materialien verwendet werden. Spezielle Beispiele für eine geeignete Siliciumquelle sind zum Beispiel kolloidale Kieselsäure oder Alkalisilicate. Beispiele für Alkaliquellen sind u.a. Natriumhydroxid und Kaliumhydroxid. Beispiele für Aluminiumquellen sind u.a. Alkalialuminate, speziell Natriumaluminat.

Als Templat werden Tetraalkylammoniumverbindungen, vorzugsweise Tetrapropylammoniumhydroxid (TPAOH) oder Tetrapropylammoniumbromid (TPABr) eingesetzt. Man kann als Templat auch Gemische aus Ammoniak oder einem organischen Amin und einer weiteren organischen Verbindung aus der Gruppe der Alkohole, vorzugsweise Butanol, verwenden.

Falls der Katalysator nach einer besonders bevorzugten erfindungsgemäßen Ausführungsform in einem CMO(Conversion of Methanol-to-Olefins)- bzw. MTP(Methanol-to-Propylene)-Verfahren verwendet werden soll, insbesondere einem Verfahren gemäß der DE 100 27 159 A1, deren diesbezügliche Offenbarung hiermit in die Beschreibung aufgenommen wird, werden die Gewichtsanteile zwischen Siliciumquelle und Aluminiumquelle so gewählt, dass kristalline Alumosilicate mit einem Si/Al-Atomverhältnis zwischen etwa 50 und 250, vorzugsweise etwa 50 und 150, insbesondere etwa 75 bis 120 erhalten werden.

Falls der fertige Katalysator nach einer weiteren besonders bevorzugten erfindungsgemäßen Ausführungsform zur Verwendung in einem OTO(Olefins-to-Olefins)-Verfahren vorgesehen ist, insbesondere einem Verfahren gemäß der DE 100 00 889 A1, deren diesbezügliche Offenbarung hiermit in die Beschreibung aufgenommen wird, werden die Gewichtsanteile zwischen Siliciumquelle und Aluminiumquelle so gewählt, dass kristalline Alumosilicate mit einem Si/Al-Atomverhältnis zwischen etwa 10 und 100, vorzugsweise zwischen etwa 20 und 65, insbesondere etwa 20 bis 50 erhalten werden.

Aus dem Reaktionsansatz wird bei erhöhter Temperatur und gegebenenfalls bei erhöhtem Druck in an sich bekannter Weise ein alkalisches Alumosilicatgel erzeugt. Die Umsetzung ist bereits bei Temperaturen unter 90°C möglich, doch werden in diesem Fall die Reaktionszeiten verhältnismäßig lang (etwa 1 Woche). Es wird deshalb vorzugsweise bei Temperaturen von 90 bis 190°C, insbesondere von 90 bis 175°C gearbeitet, bevorzugt von 100 bis 150°C, wobei sich bei Temperaturen von mehr als 100°C (unter Normalbedingungen) in Abhängigkeit von der Temperatur automatisch ein Überdruck einstellt. Das Alumosilicatgel wandelt sich im Laufe der Reaktion in ein kristallines Alumosilicat um. Wenn die Temperatur des Reaktionsansatzes höher als 190°C liegt, wird das Wachstum der Alumosilicat-Primärkristallite zu schnell und es werden leicht zu große Primärkristallite erhalten, während gleichzeitig noch Alumosilicatgel im Reaktionsansatz vorhanden ist.

Der wässrige Reaktionsansatz hat vorzugsweise einen pH-Wert von 10 bis 13. Bei einem pH-Wert von weniger als 10 verläuft die Umwandlung des Alumosilicatgels in das kristalline Alumosilicat verhältnismäßig langsam. Bei höheren pH-Werten als 13 können sich die Alumosilicatkristalle in einigen Fällen wieder auflösen.

Die Bildung der kristallinen Alumosilicat-Primärkristallite kann durch geeignete Auswahl der Siliciumquelle, der Aluminiumquelle, der Alkaliquelle und des Templats sowie durch geeignete Auswahl der Temperatur und des pH-Werts und der Rührgeschwindigkeit geregelt werden. Wesentlich ist jedoch vor allem, dass die Reaktion abgebrochen wird, wenn die erhaltenen Primärkristallite einen mittleren Durchmesser von zwischen 0,01 $\mu$m und weniger als 0,1 $\mu$m besitzen. Vorzugsweise liegt der mittlere Durchmesser der Primärkristallite im Bereich von 0,01 bis 0,06 $\mu$m. Besonders gute Resultate werden erhalten, wenn der mittlere Durchmesser der Primärkristallite im Bereich von 0,015 bis 0,05 $\mu$m liegt. Wenn der mittlere Durchmesser kleiner als 0,01 $\mu$m ist, vermindert sich sowohl die Aktivität als auch die Lebensdauer der Katalysatoren beträchtlich.

Der mittlere Durchmesser der Primärkristallite wird definiert als das (über eine Vielzahl von Kristalliten, z. B. von 10 bis 100, vorzugsweise 10 bis 20, beispielsweise 14 oder 15 gemittelte) arithmetische Mittel zwischen dem größten und dem kleinsten Durchmesser eines einzelnen Kristalliten, der anhand von rasterelektronenmikroskopischen Untersuchungen bei einer Vergrößerung von 80.000 bestimmt wird. Diese Definition hat ihre Bedeutung bei Kristalliten mit einem unregelmäßigen Kristallhabitus, z. B. bei stäbchenförmigen Kristalliten. Bei kugelförmigen bzw. annähernd kugelförmigen Kristalliten fallen der größte und der kleinste Durchmesser zusammen.

Die rasterelektronischen Untersuchen erfolgen mit einem LEO Field Emission Scanning Electron Microscope (LEO Electron Microscopy Inc., USA) anhand von Pulverproben des Katalysators, die zuvor in Aceton redispergiert, 30 Sekunden mit Ultraschall behandelt und anschließend auf einen Träger aufgebracht worden waren (Probe Current Range: 4 pA bis 10 nA). Die Messung erfolgt bei 80.000-facher Vergrößerung. Die Werte konnten bei 253.000-facher Vergrößerung bestätigt werden.

Zu diesem Zweck werden mehrere Testansätze durchgeführt. Schon nach wenigen Versuchen lassen sich die optimalen Parameter ermitteln, aufgrund derer die erforderlichen Größenbereiche der Primärkristallite erreicht werden. Ein Indiz für die Beendigung der Reaktion besteht auch darin, dass der pH-Wert des Reaktionsansatzes sprunghaft ansteigt.

Nicht in jedem Fall muss ein neuer Reaktionsansatz hergestellt werden. Vielmehr kann zur Erzeugung des Alumosilicatgels die Siliciumquelle, die Alkaliquelle, die Aluminiumquelle, das Templat und das Wasser aus den Mutterlaugen vorheriger Synthesen verwendet und durch die für die Synthese des Alumosilicatgels erforderlichen Mengen der genannten Verbindungen ergänzt werden.

Die Bildung der Alumosilicat-Primärkristallite erfolgt vorzugsweise bei einem pH-Wert zwischen 10 und 13, wobei der Reaktionsansatz gerührt wird. Auf diese Weise wird die Größenverteilung der Primärkristallite homogenisiert. Die Rührgeschwindigkeit soll aber vorzugsweise nicht mehr als 900 Umdrehungen/Minute (U/min) betragen. Bei größeren Rührgeschwindigkeiten ist der Anteil der kleineren Primärkristallite höher, was gegebenenfalls vorteilhaft ist, solange gewährleistet ist, dass der mittlere Durchmesser aller Primärkristallite mindestens 0,01 $\mu$m beträgt.

Die Primärkristallite können teilweise zu Agglomeraten vereinigt sein, die jedoch nur locker aneinander gebunden sind, wie zum Beispiel in einem Filterkuchen. Aus diesem können die Primärkristallite verhältnismäßig leicht, z.B. durch Dispergieren des Filterkuchens in einem wässrigen Medium und durch Rühren der Dispersion, wiedererhalten werden.

Die Bildung solcher Agglomerate bzw. Voragglomerate aus Primärkristalliten ist für den darauf folgenden Trennschritt bevorzugt und kann auch gezielt herbeigeführt werden, indem man dem wässrigen Reaktionsmedium ein Flockungsmittel zusetzt. Im Allgemeinen wird als Flockungsmittel eine kationische organische makromolekulare Verbindung verwendet.

Der Zusatz von Flockungsmittel erleichtert nicht nur die Abtrennung der Primärkristallite in Form von Voragglomeraten aus dem Reaktionsmedium (verbesserte Filtrierbarkeit), sondern bewirkt auch, dass die zu Voragglomeraten zusammengeschlossenen Primärkristallite hinsichtlich Größe, Struktur und Anlagerung der Primärkristallite schon weitgehend den in der folgenden Stufe gebildeten Agglomeraten gleichen.

Die Voragglomerate werden getrocknet und vorzugsweise einer Zwischenkalzinierung unterzogen, die zunächst vorzugsweise in einer inerten Atmosphäre bei etwa 200 bis 350°C, insbesondere bei etwa 250°C durchgeführt wird, wobei ein Teil des Templats desorbiert wird. Anschließend kann die Zwischenkalzinierung in einer oxidierenden Atmosphäre bei etwa 500 bis 600°C vervollständigt werden, wobei die gegebenenfalls noch vorhandene Restmenge an Templat abgebrannt wird. Im Allgemeinen werden die Voragglomerate etwa 1 bis 20 Stunden in der inerten Atmosphäre und etwa 1 bis 30 Stunden in der oxidierenden Atmosphäre zwischenkalziniert.

In Schritt (ii) wird das Produkt aus Schritt (i) zum Austausch der Alkaliionen in wässrigem Medium mit einer protonenhaltigen oder Protonen abgebenden Substanz umgesetzt. Beispielsweise kann der Ionenaustausch mit Hilfe einer verdünnten Mineralsäure, wie z.B. Salzsäure oder Schwefelsäure, oder einer organischen Säure, wie z.B. Essigsäure, durchgeführt werden. Der Ionenaustausch erfolgt vorzugsweise unter Rühren mindestens eine Stunde bei Temperaturen zwischen 25 und 100°C, wobei zumindest ein Teil der Alkaliionen in den Voragglomeraten der Primärkristallite durch Wasserstoffionen ausgetauscht wird. Falls erforderlich, kann der Ionenaustausch unter den gleichen Bedingungen wiederholt werden.

Statt mit verdünnter Säure kann der Ionenaustausch auch mit Hilfe einer Ammoniumsalzlösung unter vergleichbaren Bedingungen durchgeführt werden. In diesem Fall werden die Alkaliionen durch Ammoniumionen ausgetauscht. Wird das so erhaltene Produkt zwischenkalziniert, so wird Ammoniak entfernt, und man erhält ein Protonen enthaltendes Produkt.

In Schritt (iii) des Verfahrens wird das Protonen enthaltende Produkt (H-Zeolith) aus Schritt (b) abgetrennt (beispielsweise durch Filtration), getrocknet und erneut einer Trocknung und optional einer Kalzinierung unterzogen. Diese Kalzinierung kann bei Temperaturen von 400 bis 800°C, vorzugsweise bei etwa 600°C über einen Zeitraum von 5 bis 20 Stunden durchgeführt werden.

Bei Einhaltung der vorstehend definierten Primärkristallitgröße werden besonders gute Katalysatoren erhalten, wenn feinteiliges Aluminiumoxidhydrat in einer Menge von etwa 10 Gew.-% bis etwa 40 Gew.-%, bezogen auf das Gesamtgewicht aus Aluminiumoxidhydrat und H-Zeolith, zu dem H-Zeolithen zugegeben wird.

Durch das Vermischen des H-Zeolithen in Schritt b) mit der Vormischung aus feinteiligem Aluminiumoxidhydrat und wässrigem säurehaltigem Medium aus Schritt a) werden die Agglomerate aus Alumosilicat-Primärkristalliten durch das feinteilige Aluminiumoxidhydrat miteinander verbunden. Diese Peptisation des Aluminiumoxidhydrats bewirkt, dass die Primärkristallite bzw. die Agglomerate des Aluminosilicats durch das feinteilige Aluminiumoxidhydrat miteinander verbunden werden. Die Verbundkörper haben im Allgemeinen Abmessungen von 20 bis 1000 $\mu$m, insbesondere von 50 bis 800 $\mu$m. Auch bei diesen Werten handelt es sich um mittlere Abmessungen, die wie vorstehend angegeben definiert sind.

Das Produkt aus Schritt (b) wird in Schritt (c) einer Kalzinierung unterzogen. Diese kann allgemein bei Temperaturen zwischen etwa 500° und 850°C für 1 bis 12 Stunden durchgeführt werden. Es wurde jedoch im Rahmen der vorliegenden Erfindung auch überraschend gefunden, dass die Kalzinierung besonders vorteilhaft bei einer Temperatur im Bereich von 500 bis 700°C für 6 Stunden oder weniger, insbesondere im Bereich von 550°C bis 600°C für 1 bis 5 Stunden durchgeführt wird. Durch diese verhältnismäßig kurze Kalzinierung bei hohen Temperaturen lässt sich offenbar die Azidität der sauren Zentren des Katalysators vorteilhaft beeinflussen und zugleich die Stabilität des erfindungsgemäßen Katalysators erhöhen. Es wurde auch gefunden, dass diese vorteilhafte "verschärfte" Kalzinierung auch bei anderen Alumosilicatkatalysatoren bei Verwendung anderer (beliebiger) Aluminium, Alkali- und Siliciumquellen, beliebiger Template sowie nicht erfindungsgemäßer Bindemittel positive Effekte auf die katalytischen Eigenschaften des Katalysators auf Alumosilicatbasis zeigt.

Das so erhaltene Endprodukt kann, wie vorstehend erwähnt, besonders vorteilhaft als Katalysator in CMO- bzw. MTP- und OTO-Verfahren eingesetzt werden. Prinzipiell ist jedoch auch die Verwendung als Katalysator in anderen Kohlenstoff-Umwandlungsreaktionen, wie insbesondere Olefin-zu-Diesel-(COD-) Verfahren, Dewaxing-Verfahren, Alkylierungen, der Umwandlung von Paraffin zu aromatischen Verbindungen (CPA) sowie verwandten Reaktionen nicht ausgeschlossen.

Die vorliegende Erfindung stellt somit auch einen Katalysator zur Verfügung, der durch das oben beschriebene Verfahren erhältlich ist.

Der Aufbau des Katalysators aus Primärkristalliten, Agglomeraten und Bindemittelteilchen bestimmt auch die BET-Oberfläche, gemessen gemäß DIN 66131, die üblicherweise im Bereich von 300 bis 600 m$^2$/g liegt. Das Porenvolumen, das nach der Quecksilberporosimetrie-Methode gemäß DIN 66133 ermittelt wird, liegt für die erfindungsgemäß erhältlichen Katalysatoren vorzugsweise im Bereich von 0,31 bis 0,44 cm$^3$/g, bevorzugter im Bereich von 0,36 bis 0,44 cm$^3$/g, besonders bevorzugt im Bereich von 0,38 bis 0,44 cm$^3$/g und ganz besonders bevorzugt im Bereich von 0,41 bis 0,44 cm$^3$/g. Vorzugsweise besitzen mindestens 10%, speziell mindestens 20%, und insbesondere mindestens 60% der Poren einen Durchmesser von 14 bis 80 nm.

Die Kombination von BET-Oberfläche, Porenvolumen und Porendurchmesser stellen eine besonders geeignete Auswahl dar, um Katalysatoren mit hoher Aktivität, Selektivität und Lebensdauer zu erhalten

Die Erfindung ist durch die nachstehenden nichtbeschränkenden Beispiele näher erläutert.

**Beispiele**

Die mittlere Primärkristallitgröße wurde wie oben beschrieben mit Hilfe von rasterelektronischen Untersuchungen ermittelt.

Die mittlere Seitendruckfestigkeit wurde aus der Kraft bestimmt, die auf die Seitenfläche (längste Seite) der geformten Körper einwirkt, bis der Bruch eintritt. Dazu wurden aus einer repräsentativen Probe von Formkörpern 50 Formkörper mit einer Länge im Bereich von 5,5 bis 6,5 mm ausgewählt und einzeln vermessen. Die Formkörper waren rissfrei und gerade geformt. Ein geformter Körper wurde zwischen zwei Messbacken (eine bewegliche und eine unbewegliche) gelegt. Die bewegliche Messbacke wurde dann gleichmäßig auf den geformten Körper zu bewegt, bis der Bruch des Formkörpers eintrat. Der Bruchmesswert in Kilopond (kp), gemessen mit einem Messgerät der Firma Schleuniger, wurde durch die Länge des Formkörpers geteilt, um die Seitendruckfestigkeit des Formkörpers zu erhalten. Aus 50 Einzelmessungen wurde dann die mittlere Seitendruckfestigkeit bestimmt.

Die Messung des Porenvolumens nach der Quecksilberporosimetrie-Methode und die Berechnung des Porendurchmessers erfolgten gemäß DIN 66133.

Der mittlere Methanol-Umsatz wurde wie in nachstehendem Anwendungsbeispiel 1 beschrieben gemessen.

**Referenzbeispiel 1:** Herstellung eines H-Zeolithen mit einer mittleren Primärkristallitgröße von 0,03 $\mu$m

Eine Reaktionsmischung wurde durch inniges Mischen zweier Lösungen bei Raumtemperatur in einem 40-Liter-Autoklaven hergestellt. Die beiden Lösungen wurden als Lösung A und Lösung B bezeichnet. Die Lösung A wurde hergestellt, indem in 11 kg deionisiertem Wasser 2218 g Tetrapropylammoniumbromid gelöst wurden. In diese Lösung wurden 5000 g einer handelsüblichen Kieselsäure eingetragen. Die Lösung B wurde hergestellt, indem in 5,5 Liter deionisiertem Wasser 766 g NaOH und anschließend 45,6 g $NaAlO_2$ gelöst wurden. Die noch warme Lösung B wurde zur Lösung A gegeben. Der Autoklav wurde dann geschlossen und unter Rühren bei etwa 60 U/min. auf die Reaktionstemperatur gebracht. Die Reaktion wurde beendet, sobald der mittlere Teilchendurchmesser der Primärkristallite bei 0,03 $\mu$m lag. Nach dem Abkühlen wurde der Autoklav geöffnet, das Produkt dem Reaktionskessel entnommen und filtriert. Der Filterkuchen wurde in etwa 40 Liter deionisiertem Wasser aufgeschlämmt, mit etwa 5 Liter einer 0,4 gew.-%igen wässrigen Suspension eines handelsüblichen Flockungsmittels versetzt und nach dem Rühren und Absetzen der Voragglomerate des Feststoffes dekantiert. Der beschriebene Waschprozess wurde wiederholt, bis das Waschwasser einen pH-Wert von 7 bis 8 und eine Br-Konzentration von weniger als 1 ppm hatte. Die Aufschlämmung, in der Voragglomerate von Primärkristalliten zu erkennen waren, die offenbar durch das Flockungsmittel zusammengehalten wurden, wurde filtriert. Der Filterkuchen wurde anschließend bei 120°C 12 Stunden getrocknet.

Der getrocknete Filterkuchen wurde mit einem handelsüblichen Granulator auf eine Korngröße von 2 mm zerkleinert.

Das Granulat wurde mit einer Aufheizrate von 1°C/Minute unter Stickstoff (1000 Nl/h) auf 350°C gebracht und bei 350°C 15 Stunden unter Stickstoff (1000 Nl/h) kalziniert. Dann wurde die Temperatur mit einer Aufheizrate von 1°C/Minute auf 540°C erhöht, und das Granulat wurde 24 Stunden bei dieser Temperatur an Luft kalziniert, um das restliche Tetrapropylammoniumbromid abzubrennen.

Der kalzinierte Na-Zeolith wurde in der 5-fachen Menge einer 1-molaren wässrigen HCl-Lösung suspendiert und auf 80°C gebracht. Bei dieser Temperatur wurde eine Stunde gerührt. Dann wurde etwa 1 Liter einer 0,4 gew.-%igen Suspension des Flockungsmittels zugegeben, und die überstehende Säure wurde nach Absetzen des Feststoffes abdekantiert. Der so beschriebene Vorgang wurde noch einmal wiederholt. Der Feststoff wurde in etwa 10 Waschvorgängen jeweils in 60 Liter deionisiertem Wasser unter Rühren suspendiert und mit durchschnittlich 100 ml einer 0,4 gew.-%igen Suspension des Flockungsmittels versetzt. Nach dem Absitzen des Zeolithen wurde die überstehende Lösung dekantiert. Als der Gehalt an Cl$^-$ im Waschwasser < 5 ppm war, wurde die Suspension abfiltriert und bei 120°C 15 Stunden getrocknet.

Der getrocknete H-Zeolith wurde mit einem handelsüblichen Granulator auf 2 mm zerkleinert und unter Luft mit einer Aufheizrate von 1°C/Minute auf 540°C gebracht und bei dieser Temperatur unter Luft 10 Stunden kalziniert.

**Beispiel 1:** Katalysator 1

In einem handelsüblichen Mischkneter wurden 61 kg entsalztes Wasser und 57 kg eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurden langsam 69 kg einer Lösung bestehend aus 39 kg 57,2 %iger Salpetersäure und 30 kg entsalztes Wasser gegeben. Diese Mischung wurde 60 Minuten bis zur Plastifizierung und Homogenisierung geknetet. Anschließend wurden 235 kg des kalzinierten H-Zeolithen aus Referenzbeispiel 1, der auf einer handelsüblichen Mühle auf eine Korngröße von kleiner etwa 500 $\mu$m gemahlen war, zugegeben. Es wurde weitere 30 Minuten gemischt und zur Verbesserung der Konsistenz der Masse noch etwa 25 kg Wasser zugegeben.

Nach Einmischen von 20 kg Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 1,5 kp/mm (14,7 N/mm) Quecksilberporenvolumen: 0,31 cm$^3$/g
Mittlerer Methanol-Umsatz a): 95,2%

a) gemittelt über 200 - 400 hours-on-stream (HOS)

**Beispiel 2:** Katalysator 2

In einem handelsüblichen Mischkneter wurden 54 kg entsalztes Wasser und 50 kg eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurden langsam 60 kg einer Lösung bestehend aus 33 kg 58,2 %iger Salpetersäure und 27 kg entsalztes Wasser gegeben. Diese Mischung wurde 60 Minuten bis zur Plastifizierung und Homogenisierung geknetet. Anschließend wurden 200 kg des kalzinierten H-Zeolithen aus Referenzbeispiel 1, der auf einer handelsüblichen Mühle auf eine Korngröße von kleiner etwa 500 $\mu$m gemahlen war, zugegeben. Es wurde weitere 30 Minuten gemischt und zur Verbesserung der Konsistenz der Masse noch etwa 18 kg Wasser zugegeben. Nach Einmischen von 17 kg Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 1,5 kp/mm (14,7 N/mm) Quecksilberporenvolumen: 0,33 cm$^3$/g
Mittlerer Methanol-Umsatz a): 95,1%

a) gemittelt über 200 - 400 hours-on-stream (HOS)

**Beispiel 3:** Katalysator 3

In einem handelsüblichen Doppel-Z-Kneter wurden 198 g entsalztes Wasser und 145 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurde langsam 130 g einer Lösung bestehend aus 31 g 52,2 %iger Salpetersäure und 99 g entsalztes Wasser gegeben. Diese Mischung wurde 60 Minuten bis zur Plastifizierung und Homogenisierung geknetet. Anschließend wurden 600 g des kalzinierten H-Zeolithen aus Referenzbeispiel 1, der auf einer handelsüblichen Mühle auf eine Korngröße von kleiner etwa 500 $\mu$m gemahlen war, zugegeben. Es wurde weitere 30 Minuten gemischt und zur Verbesserung der Konsistenz der Masse noch etwa 11 g Wasser zugegeben. Nach Einmischen von 50 g Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,8 kp/mm (7,8 N/mm) Quecksilberporenvolumen: 0,38 cm$^3$/g
Mittlerer Methanol-Umsatz a): 97,6%

a) gemittelt über 200 - 400 hours-on-stream (HOS)

**Beispiel 4:** Katalysator 4

In einem handelsüblichen Doppel-Z-Kneter wurden 104 g entsalztes Wasser und 102 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurde langsam 127 g einer Lösung bestehend aus 75 g 52,5 %iger Salpetersäure und 52 g entsalztes Wasser gegeben. Diese Mischung wurde 60 Minuten bis zur Plastifizierung und Homogenisierung geknetet. Anschließend wurden 400 g des kalzinierten H-Zeolithen aus Referenzbeispiel 1, der auf einer handelsüblichen Mühle auf eine Korngröße von kleiner etwa 500 $\mu$m gemahlen war, zugegeben. Es wurde weitere 30 Minuten gemischt und zur Verbesserung der Konsistenz der Masse noch etwa 150 g Wasser zugegeben. Nach Einmischen von 34 g Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,8 kp/mm (7,8 N/mm) Quecksilberporenvolumen: 0,41 cm$^3$/g
Mittlerer Methanol-Umsatz a): 99,1%
Mittlere Olefinselektivität a):

Propylen: 43,7%
Butene: 21,6%
C$_{2-4}$-Olefine: 72,0%

a) gemittelt über 200 - 400 hours-on-stream (HOS)

**Beispiel 5:** Katalysator 5

In einem handelsüblichen Doppel-Z-Kneter wurden 25 kg entsalztes Wasser und 23 kg eines handelsüblichen, zur Peptisation fähigen Aluminiumoxidhydrats mit einem Korngrößenspektrum von 91 Vol.-% ≤ 90 μm; 51 Vol.-% ≤ 45 μm und 27 Vol.-% ≤ 25 μm gemischt. Zu dieser Mischung wurde langsam 28 kg einer Lösung bestehend aus 16 kg 57,2 %iger Salpetersäure und 12 kg entsalztes Wasser gegeben. Diese Mischung wurde 60 Minuten bis zur Plastifizierung und Homogenisierung geknetet. Anschließend wurden 94 kg des kalzinierten H-Zeolithen aus Referenzbeispiel 1, der auf einer handelsüblichen Mühle auf eine Korngröße von kleiner etwa 500 μm gemahlen war, und 2 kg Mikrohohlkugeln als Ausbrennstoff zugegeben. Es wurde weitere 30 Minuten gemischt und zur Verbesserung der Konsistenz der Masse noch etwa 10 kg Wasser zugegeben. Nach Einmischen von 8 kg Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,9 kp/mm (8,8 N/mm) Quecksilberporenvolumen: 0,44 cm$^3$/g
Mittlerer Methanol-Umsatz a): 99,0%

a) gemittelt über 200 - 400 hours-on-stream (HOS)

**Beispiel 6 (Vergleichsbeispiel):** Katalysator 6

750 g des kalzinierten H-Zeolithen aus Referenzbeispiel 1 wurden mit Hilfe einer Labormühle auf eine Korngröße von kleiner etwa 500 μm gemahlen und in einem handelsüblichen Doppel-Z-Kneter mit 220 g eines handelsüblichen, zur Peptisation fähigen Aluminiumoxidhydrats mit einem Korngrößenspektrum von 98 Vol.-% ≤ 90 μm; 95 Vol.-% ≤ 45 μm und 55 Vol.-% ≤ 25 μm 15 Minuten trocken gemischt. Zu dieser Mischung wurden langsam 487,5 g einer 1,5 gew.-%igen wässrigen Essigsäurelösung und 50 g Steatitöl gegeben. Nach Zugabe von 55 g einer 1,5 gew.-%igen wässrigen Essigsäurelösung wurde 30 Minuten nachgeknetet und die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,6 kp/mm (5,9 N/mm)
Quecksilberporenvolumen: 0,46 cm$^3$/g

Aufgrund der geringen Seitendruckfestigkeit ließ sich der mittlere Methanol-Umsatz nicht messen; er lässt sich jedoch auf über 99 % abschätzen

**Beispiel 7 (Vergleichsbeispiel):** Katalysator 7

700 g des kalzinierten H-Zeolithen aus Referenzbeispiel 1 wurden mit Hilfe einer Labormühle auf eine Korngröße von kleiner etwa 500 μm gemahlen und in einem handelsüblichen Doppel-Z-Kneter mit 303 g eines handelsüblichen, zur Peptisation fähigen Aluminiumoxidhydrats mit einem Korngrößenspektrum von 91 Vol.-% ≤ 90 μm; 51 Vol.-% ≤ 45 μm und 27 Vol.-% ≤ 25 μm und 105 g Paraffinwachs 15 Minuten trocken gemischt. Zu dieser Mischung wurden langsam 245 g entsalztes Wasser und 127 g einer 25 gew.-%igen wässrigen Zitronensäurelösung und weitere 135 g entsalztes Wasser gegeben. Nach Zugabe von 56 g Steatitöl wurde 5 Minuten nachgeknetet und die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,7 kp/mm (6,9 N/mm)

Quecksilberporenvolumen: 0,47 cm$^3$/g

Aufgrund der geringen Seitendruckfestigkeit ließ sich der mittlere Methanol-Umsatz nicht messen; er lässt sich jedoch auf über 99 % abschätzen

**Beispiel 8 (Vergleichsbeispiel):** Katalysator 8

700 g des kalzinierten H-Zeolithen aus Referenzbeispiel 1 wurden mit Hilfe einer Labormühle auf eine Korngröße von kleiner etwa 500 $\mu$m gemahlen und in einem handelsüblichen Doppel-Z-Kneter mit 302 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m und 136 g Paraffinwachs 15 Minuten trocken gemischt. Zu dieser Mischung wurden langsam 245 g entsalztes Wasser und 127 g einer 25 gew.-%igen wässrigen Zitronensäurelösung und weitere 206 g entsalztes Wasser gegeben. Nach Zugabe von 56 g Steatitöl wurde 5 Minuten nachgeknetet und die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 0,4 kp/mm (3,9 N/mm)
Quecksilberporenvolumen: 0,51 cm$^3$/g

Aufgrund der geringen Seitendruckfestigkeit ließ sich der mittlere Methanol-Umsatz nicht messen; er lässt sich jedoch auf über 99 % abschätzen.

**Beispiel 9 (Vergleichsbeispiel):** Katalysator 9

750 g des kalzinierten H-Zeolithen aus Referenzbeispiel 1 wurden mit Hilfe einer Labormühle auf eine Korngröße von kleiner etwa 500 $\mu$m gemahlen und in einem handelsüblichen Doppel-Z-Kneter mit 225 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m 15 Minuten trocken gemischt. Zu dieser Mischung wurden langsam 583 g einer 1,5 gew.-%igen wässrigen Salpetersäurelösung und 50 g Steatitöl gegeben. 30 Minuten nachgeknetet und die plastifizierte Masse in einem handelsüblichen Extruder zu Formkörpern mit einem Durchmesser von etwa 3 mm und einer Länge von etwa 6 mm extrudiert. Die Formkörper wurden anschließend 16 Stunden bei 120°C getrocknet und 5 Stunden bei 600°C kalziniert.

Mittlere Seitendruckfestigkeit: 2,0 kp/mm (19,5 N/mm)
Quecksilberporenvolumen: 0,32 cm$^3$/g

**Beispiel 10:** Katalysator 10

In einem handelsüblichen Doppel-Z-Kneter wurden 175 g entsalztes Wasser und 170 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurden langsam 199 g einer Lösung bestehend aus 64 g 99-100 %iger Essigsäure und 135 g entsalztes Wasser gegeben. Diese Mischung wurde 60 Minuten bis zur Plastifizierung und Homogenisierung geknetet. Anschließend wurden 700 g des kalzinierten H-Zeolithen aus Referenzbeispiel 1, der auf einer handelsüblichen Mühle auf eine Korngröße von kleiner etwa 500 $\mu$m gemahlen war, zugegeben. Es wurde weitere 30 Minuten gemischt und zur Verbesserung der Konsistenz der Masse noch etwa 80 g Wasser zugegeben. Nach Einmischen von 59 g Steatitöl und 10 Minuten Mischen wurde die plastifizierte Masse in einem handelsüblichen Extruder zu Formkörpern mit einem Durchmesser von etwa 3 mm und einer Länge von etwa 6 mm extrudiert. Die Formkörper wurden anschließend 16 Stunden bei 120°C getrocknet und 5 Stunden bei 600°C kalziniert.

Mittlere Seitendruckfestigkeit: 0,90 kp/mm (8,8 N/mm)
Quecksilberporenvolumen: 0,36 cm$^3$/g

**Beispiel 11:** Katalysator 11

In einem handelsüblichen Mischkneter wurden 910 g entsalztes Wasser und 889 g eines handelsüblichen, zur Peptisation fähigen Aluminiumoxidhydrats mit einem Korngrößenspektrum von 91 Vol.-% $\leq$ 90 $\mu$m; 51 Vol.-% $\leq$ 45 $\mu$m und 27 Vol.-% $\leq$ 25 $\mu$m gemischt. Zu dieser Mischung wurde langsam 1108 g einer Lösung bestehend aus 653 g 52,5 %iger Salpetersäure und 455 g entsalztes Wasser gegeben. Diese Mischung wurde 60 Minuten bis zur Plastifizierung und Homogenisierung geknetet. Anschließend wurden 3000 g des kalzinierten H-Zeolithen aus Referenzbeispiel 1, der auf einer handelsüblichen Mühle auf eine Korngröße von kleiner etwa 500 μm gemahlen war, zugegeben. Es wurde weitere 30 Minuten gemischt und zur Verbesserung der Konsistenz der Masse noch etwa 350 g Wasser zugegeben. Nach Einmischen von 252 g Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 1,3 kp/mm (12,7 N/mm)
Quecksilberporenvolumen: 0,36 cm$^3$/g
Mittlerer Methanol-Umsatz a): 97,2%
Mittlere Olefinselektivität a):

Propylen: 43,2%
Butene: 20,3%
C$_{2-4}$-Olefine: 70,0%

a) gemittelt über 200 - 400 hours-on-stream (HOS)

**Beispiel 12:** Katalysator 12

In einem handelsüblichen Mischkneter wurden 1869 g entsalztes Wasser und 1827 g eines handelsüblichen, zur Peptisation fähigen Aluminiumoxidhydrats mit einem Korngrößenspektrum von 91 Vol.-% ≤ 90 μm; 51 Vol.-% ≤ 45 μm und 27 Vol.-% ≤ 25 μm gemischt. Zu dieser Mischung wurde langsam 2276 g einer Lösung bestehend aus 1342 g 52,5 %iger Salpetersäure und 934 g entsalztem Wasser gegeben. Diese Mischung wurde 60 Minuten bis zur Plastifizierung und Homogenisierung geknetet. Zu 2986 g der erhaltenen plastifizierten Masse wurden anschließend 3000 g des kalzinierten H-Zeolithen aus Referenzbeispiel 1, der auf einer handelsüblichen Mühle auf eine Korngröße von kleiner etwa 500 μm gemahlen war, zugegeben. Es wurde weitere 30 Minuten gemischt und zur Verbesserung der Konsistenz der Masse noch etwa 200 g Wasser zugegeben. Nach Einmischen von 252 g Steatitöl und 10 Minuten Mischen wurde die plastifizierte Masse in einem handelsüblichen Extruder zu Formkörpern mit einem Durchmesser von etwa 3 mm und einer Länge von etwa 5 mm extrudiert. Die Formkörper wurden anschließend 16 Stunden bei 120°C getrocknet und 5 Stunden bei 600°C kalziniert.

Mittlere Seitendruckfestigkeit: 1,4 kp/mm (13,7 N/mm)
Quecksilberporenvolumen: 0,31 cm$^3$/g
Mittlerer Methanol-Umsatz a): 96,1%
Mittlere Olefinselektivität a):

Propylen: 42,2%
Butene: 19,4%
C$_{2-4}$-Olefine: 68,3%

a) gemittelt über 200 - 400 hours-on-stream (HOS)

**Anwendungsbeispiel 1**

Dieses Anwendungsbeispiel zeigt anhand katalytischer Daten des CMO-Verfahrens (Conversion of Methanol-to-Olefins-Verfahren) in einem isothermen Festbettreaktor die Vorteile des erfindungsgemäßen Katalysators.
Alle getesteten Katalysatoren wurden 48 Stunden mit Wasserdampf behandelt, bevor das Methanol/Wasser-Gemisch aufgegeben wurde. Anschließend wurde das Feed Methanol/Wasser mit einer WHSV von 3 (kg/(kg·h), d.h. drei Kilogramm Gesamtfeed pro Kilogramm Katalysator und pro Stunde bei einem Druck von 1 bar zur Konversion von Methanol über den CMO-Katalysator in einem isothermen Festbettrohrreaktor geleitet. Der Methanol-Gehalt der Gasphase und der flüssigen Phase am Ausgang des CMO-Katalysator-Reaktors wurden mit gaschromatographischen Analyseverfahren bestimmt. Tabelle 1 zeigt die katalytischen Eigenschaften der untersuchten Katalysatoren im isothermen Reaktor bei folgenden Bedingungen: T$^R_{Aus}$ (Temperatur des Reaktors am Auslass) = 450 °C; Belastung: WHSV = 1 h$^{-1}$ (kg Methanol/kg Katalysator und Stunde), Gewichtsverhältnis (MeOH:H$_2$O) = 1:2, für 200 - 400 HOS (hours-on-stream).
Beim Vergleich der katalytischen Eigenschaften (Tabelle 1) wird deutlich, dass die erfindungsgemäßen Katalysatoren einen hohen MeOH-Umsatz bei gleichzeitig hoher mittlerer Seitendruckfestigkeit aufweisen. Katalysator 6, der dem Katalysator von Beispiel 1 der EP 1 424 128 entspricht, weist zwar einen hohen MeOH-Umsatz auf, besitzt jedoch eine Seitendruckfestigkeit, die mit 0,6 kp/mm unter dem Schwellwert von 0,8 kp/mm als akzeptable Mindest-Seitendruckfestigkeit liegt. Die Katalysatoren 7 und 8 besitzen ebenfalls inakzeptable Seitendruckfestigkeiten. Hier wurde der mittlere Methanol-Umsatz nicht bestimmt, um ein Verstopfen des Reaktors durch einen auseinanderbrechenden Katalysator zu vermeiden.

**Tabelle 1**

| Katalysator | Porenvolumen [cm$^3$/g] | Mittlerer MeOH-Umsatz (200-400 HOS) [%] | Mittlere Seitendruckfestigkeit [kp/mm] |
|---|---|---|---|
| 1 | 0,31 | 95,2 | 1,5 |
| 2 | 0,33 | 95,1 | 1,5 |
| 3 | 0,38 | 97,6 | 0,8 |
| 4 | 0,41 | 99,1 | 0,8 |
| 5 | 0,44 | 99,0 | 0,9 |
| 6 (Vergleich) | 0,46 | > 99*1 | 0,6 |
| 7 (Vergleich) | 0,47 | > 99*1 | 0,7 |
| 8 (Vergleich) | 0,51 | > 99*1 | 0,4 |
| *1: geschätzt | | | |

Die Katalysatoren können nach Beendigung eines ersten Zyklus regeneriert werden, indem zunächst der MeOH-Strom abgestellt wird. Dann wird Stickstoff zum Verdrängen des restlichen MeOH eingespeist. Schließlich wird dem Stickstoff langsam Sauerstoff in allmählich höher werdenden Konzentrationen zugesetzt, um den auf den Katalysatoren abgeschiedenen Kohlenwasserstoff abzubrennen. Die Temperatur der Katalysatoren wird hierbei üblicherweise unter 480 °C gehalten. Die Regenerierung der Katalysatoren ist beendet, wenn der Sauerstoffgehalt des Stickstoffstromes am Eingang und am Ausgang des Katalysatorbettes der gleiche ist.

**Anwendungsbeispiel 2**

Dieses Anwendungsbeispiel dient dazu, die Selektivität der erfindungsgemäßen Katalysatoren im CMO-Verfahren zu bestimmen.

Die Katalysatoren wurden auf die gleiche Weise wie in Anwendungsbeispiel 1 beschrieben vorbehandelt und getestet. Die Zusammensetzung der Gasphase und der flüssigen Phase am Ausgang des CMO-Katalysator-Reaktors wurden mit gaschromatographischen Analyseverfahren bestimmt.

Die Selektivität $S_i$ resultiert aus dem molaren Kohlenstoffanteil der Komponente i bezogen auf den umgesetzten Kohlenstoff:

$$S_i = \frac{\dot{n}_{i\,aus} \cdot \varepsilon_i^C}{\dot{n}_{MeOH\,ein} \cdot \varepsilon_{MeOH}^C - \dot{n}_{MeOH\,aus} \cdot \varepsilon_{MeOH}^C}$$

Um den Einfluss der Kohlenstoffbilanz auszuschließen, wurde die Selektivität wie folgt auf 100% normiert:

$$\overline{S}_i = 100\% * \frac{\dot{n}_{i\,aus} \cdot \varepsilon_i^C}{\sum_{i}^{i=N} \dot{n}_{i\,aus} \cdot \varepsilon_i^C}$$

Für diese Berechnung wurde Dimethylether nicht als Produkt berücksichtigt.

$S_i$ : Selektivität der Komponente i
$\overline{S}_i$ : Normierte Selektivität der Komponente i

$\varepsilon_i^C$ : Anzahl der Kohlenstoffatome der Komponente i

$\dot{n}_i$ : Stoffmengestrom der Komponente i

Tabelle 2 zeigt den Methanolumsatz und die Olefinselektivität der untersuchten Katalysatoren im isothermen Reaktor unter denselben Bedingungen wie in Anwendungsbeispiel 1 beschrieben.

**Tabelle 2**

| Katalysator | Porenvolumen [cm³/g] | Mittlerer MeOH-Umsatz (200-400 HOS) [%] | Mittlere Seitendruckfestigkeit [kp/mm] | Mittlere Olefinselektivität | | |
|---|---|---|---|---|---|---|
| | | | | Propylen (200-400 HOS) [%] | Butylene (200-400 HOS) [%] | $C_{2-4}$-olefine (200-400 HOS) [%] |
| 4 | 0,41 | 99,1 | 0,8 | 43,7 | 21,6 | 72,0 |
| 11 | 0,36 | 97,2 | 1,3 | 43,2 | 20,3 | 70,0 |
| 12 | 0,31 | 96,1 | 1,4 | 42,2 | 19,4 | 68,3 |

Beim Vergleich der katalytischen Eigenschaften von Tabelle 2 wird deutlich, dass alle erfindungsgemäßen Katalysatoren 4, 11 und 12 hohe mittlere Olefinselektivitäten aufweisen. Katalysator 4, der das größte Porenvolumen besitzt, zeigt dabei die beste katalytische Leistung.

**Patentansprüche**

1. Verfahren zur Herstellung eines Katalysators auf der Basis von kristallinem Alumosilicat vom Pentasil-Typ mit einem Si/Al-Atomverhältnis zwischen 10 und 100, umfassend die Schritte:

    (a) Behandeln von Aluminiumoxidhydrat mit einem wässrigen säurehaltigen Medium,
    (b) Vermischen des mit wässrigem säurehaltigem Medium behandelten Aluminiumoxidhydrats aus Schritt (a) mit einem H-Zeolithen mit einem mittleren Durchmesser der Primärkristallite von zwischen 0,01 $\mu$m und weniger als 0,1 $\mu$m und
    (c) Kalzinieren der in Schritt (b) erhaltenen Mischung, wobei mindestens 95 Vol.-% der Teilchen des Aluminiumoxidhydrats, auf den mittleren Durchmesser bezogen, kleiner oder gleich 100 $\mu$m ist.

2. Verfahren gemäß Anspruch 1, wobei das wässrige säurehaltige Medium eine wässrige anorganische Säure, wie Schwefelsäure oder Salpetersäure, insbesondere Salpetersäure ist.

3. Verfahren gemäß Anspruch 1, wobei das säurehaltige Medium eine wässrige organische Säure, wie Essigsäure, Zitronensäure, Ameisensäure oder Oxalsäure, insbesondere Essigsäure oder Zitronensäure ist.

4. Verfahren gemäß einem der vorhergehenden Ansprüche, wobei in Schritt (a) das wässrige säurehaltige Medium zu dem Aluminiumoxidhydrat in einer Menge zugegeben wird, so dass sich eine Säurekonzentration von 0,005 bis 2,5 mol H$^+$/mol Al$_2$O$_3$, vorzugsweise 0,01 bis 1,5 mol H$^+$/mol Al$_2$O$_3$ und insbesondere 0,05 bis 1,0 mol H$^+$/mol Al$_2$O$_3$ ergibt.

5. Verfahren gemäß einem der vorhergehenden Ansprüche, wobei mindestens 97 Vol.-% der Teilchen des Aluminiumoxidhydrats, auf den mittleren Durchmesser bezogen, kleiner oder gleich 100 $\mu$m ist.

6. Verfahren gemäß einem der Ansprüche 1 bis 4, wobei das Aluminiumoxidhydrat ein Korngrößenspektrum von

    91 Vol.-% $\leq$ 90 $\mu$m
    51 Vol.-% $\leq$ 45 $\mu$m
    27 Vol.-% $\leq$ 25 $\mu$m

jeweils bezogen auf den mittleren Teilchendurchmesser aufweist.

7. Verfahren gemäß einem der vorhergehenden Ansprüche, wobei das Aluminiumoxidhydrat in einer Menge von 10 Gew.-% bis 40 Gew.-% Aluminiumoxid, bezogen auf das Gesamtgewicht des Endproduktes, vorliegt.

8. Verfahren gemäß einem der vorhergehenden Ansprüche, wobei die Kalzinierung bei einer Temperatur im Bereich von 500 bis 700°C für 6 Stunden oder weniger, vorzugsweise im Bereich von 550°C bis 600°C für 1 bis 5 Stunden, erfolgt.

9. Verfahren gemäß einem der vorhergehenden Ansprüche, wobei der H-Zeolith erhältlich ist durch ein Verfahren, umfassend die folgenden Schritte:

(i) Herstellen von Alumosilicat-Kristalliten mit einem mittleren Durchmesser von größer oder gleich 0,01 $\mu$m bis weniger als 0,1 $\mu$m durch Umsetzen einer Siliziumquelle, einer Aluminiumquelle, einer Alkaliquelle und einem Templat bei einer Reaktionstemperatur von größer oder gleich 90°C,
(ii) Abtrennen und gegebenenfalls Zwischenkalzinieren der Kristallite aus Schritt (i),
(iii) Umsetzen der Alumosilicat-Kristallite aus Schritt (ii) mit einer Ionenaustauschverbindung,
(iv) Abtrennen und gegebenenfalls Kalzinieren des aus Schritt (iii) erhaltenen Produkts.

10. Katalysator, erhältlich durch ein Verfahren gemäß einem der Ansprüche 1 bis 9.

11. Katalysator gemäß Anspruch 10, dessen Porenvolumen, bestimmt nach der Quecksilberporosimetrie, 0,31 bis 0,44 cm$^3$/g, bevorzugt 0,38 bis 0,44 cm$^3$/g und besonders bevorzugt 0,41 bis 0,44 cm$^3$/g beträgt.

12. Katalysator gemäß Anspruch 10 oder 11, dessen mittlere Seitendruckfestigkeit 7,8 N/mm oder höher, insbesondere 7,8 bis 14,7 N/mm ist.

13. Katalysator gemäß einem der Ansprüche 10 bis 12, wobei der Katalysator in einem Methanol-zu-Olefin-Verfahren einen mittleren Methanol-Umsatz, gemittelt über 200-400 hours-on-stream, von 96 % oder mehr, insbesondere von 97 % besitzt.

14. Katalysator gemäß einem der Ansprüche 10 bis 13, wobei der Katalysator in einem Methanol-zu-Olefin-Verfahren einen Selektivität bei der Herstellung von C$_{2-4}$-Olefinen, gemittelt über 200-400 hours-on-stream, von 65,0 % oder mehr, vorzugsweise von 68,3% oder mehr, insbesondere von 72,0% oder mehr aufweist.

15. Verwendung eines Katalysators gemäß einem der Ansprüche 10 bis 14 in einem Methanol-zu-Olefin-Verfahren, oder eines Katalysators gemäß einem der Ansprüche 10 bis 12 in einem Olefin-zu-Olefin-Verfahren.

**Claims**

1. Process for producing a catalyst based on crystalline aluminosilicate of the pentasil type having an Si/Al atomic ratio in the range from 10 to 100, comprising the steps:

(a) treatment of aluminium oxide hydrate with an aqueous acid-containing medium,
(b) mixing of the aluminium oxide hydrate which has been treated with aqueous acid-containing medium from step (a) with an H-zeolite having an average diameter of the primary crystallites of from 0.01 $\mu$m to less than 0.1 $\mu$m and
(c) calcination of the mixture obtained in step (b), where at least 95% by volume of the particles of the aluminium oxide hydrate is, based on the average diameter, less than or equal to 100 $\mu$m.

2. Process according to Claim 1, wherein the aqueous acid-containing medium is an aqueous inorganic acid such as sulfuric acid or nitric acid, in particular nitric acid.

3. Process according to Claim 1, wherein the acid-containing medium is an aqueous organic acid such as acetic acid, citric acid, formic acid or oxalic acid, in particular acetic acid or citric acid.

4. Process according to any of the preceding claims, wherein, in step (a), the aqueous acid-containing medium is added to the aluminium oxide hydrate in such an amount that an acid concentration of from 0.005 to 2.5 mol of H$^+$/mol of Al$_2$O$_3$, preferably from 0.01 to 1.5 mol of H$^+$/mol of Al$_2$O$_3$ and in particular from 0.05 to 1.0 mol of H$^+$/mol

of $Al_2O_3$, is obtained.

5. Process according to any of the preceding claims, wherein at least 97% by volume of the particles of the aluminium oxide hydrate is, based on the average diameter, less than or equal to 100 $\mu$m.

6. Process according to any of Claims 1 to 4, wherein the aluminium oxide hydrate has a grain size spectrum of

    91% by volume $\leq$ 90 $\mu$m
    51% by volume $\leq$ 45 $\mu$m
    27% by volume $\leq$ 25 $\mu$m

    in each case based on the average particle diameter.

7. Process according to any of the preceding claims, wherein the aluminium oxide hydrate is present in an amount of from 10% by weight to 40% by weight of aluminium oxide, based on the total weight of the end product.

8. Process according to any of the preceding claims, wherein the calcination is carried out at a temperature in the range from 500 to 700°C for 6 hours or less, preferably in the range from 550°C to 600°C for from 1 to 5 hours.

9. Process according to any of the preceding claims, wherein the H-zeolite is obtainable by a process comprising the following steps:

    (i) production of aluminosilicate crystallites having an average diameter of from greater than or equal to 0.01 $\mu$m to less than 0.1 $\mu$m by reaction of a silicon source, an aluminium source, a source of alkali and a template at a reaction temperature of greater than or equal to 90°C,
    (ii) isolation and optionally intermediate calcination of the crystallites from step (i),
    (iii) reaction of the aluminosilicate crystallites from step (ii) with an ion exchange compound,
    (iv) isolation and optionally calcination of the product obtained from step (iii).

10. Catalyst obtainable by a process according to any of Claims 1 to 9.

11. Catalyst according to Claim 10 whose pore volume, determined by mercury porosimetry, is from 0.31 to 0.44 cm$^3$/g, preferably from 0.38 to 0.44 cm$^3$/g and particularly preferably from 0.41 to 0.44 cm$^3$/g.

12. Catalyst according to Claim 10 or 11 whose average lateral compressive strength is 7.8 N/mm or above, in particular from 7.8 to 14.7 N/mm.

13. Catalyst according to any of Claims 10 to 12, wherein the catalyst has, in a methanol-to-olefin process, an average methanol conversion, averaged over 200-400 hours-on-stream, of 96% or more, in particular 97%.

14. Catalyst according to any of Claims 10 to 13, wherein the catalyst has, in a methanol-to-olefin process, a selectivity in the preparation of C$_{2-4}$-olefins, averaged over 200-400 hours-on-stream, of 65.0% or more, preferably 68.3% or more, in particular 72.0% or more.

15. Use of a catalyst according to any of Claims 10 to 14 in a methanol-to-olefin process or of a catalyst according to any of Claims 10 to 12 in an olefin-to-olefin process.

**Revendications**

1. Procédé de fabrication d'un catalyseur à base d'alumosilicate cristallin de type pentasil ayant un rapport atomique Si/Al compris entre 10 et 100, comprenant les étapes suivantes :

    (a) le traitement d'oxyhydrate d'aluminium avec un milieu aqueux contenant un acide,
    (b) le mélange de l'oxyhydrate d'aluminium traité avec un milieu aqueux contenant un acide de l'étape (a) avec une zéolithe H ayant un diamètre moyen des cristallites primaires compris entre 0,01 $\mu$m et moins de 0,1 pm, et
    (c) la calcination du mélange obtenu à l'étape (b), au moins 95 % en volume des particules de l'oxyhydrate d'aluminium ayant une taille inférieure ou égale à 100 pm, en termes de diamètre moyen.

**2.** Procédé selon la revendication 1, dans lequel le milieu aqueux contenant un acide est un acide inorganique aqueux, tel que l'acide sulfurique ou l'acide nitrique, notamment l'acide nitrique.

**3.** Procédé selon la revendication 1, dans lequel le milieu contenant un acide est un acide organique aqueux, tel que l'acide acétique, l'acide citrique, l'acide formique ou l'acide oxalique, notamment l'acide acétique ou l'acide citrique.

**4.** Procédé selon l'une quelconque des revendications précédentes, dans lequel, à l'étape (a), le milieu aqueux contenant un acide est ajouté à l'oxyhydrate d'aluminium en une quantité telle qu'une concentration en acide de 0,005 à 2,5 moles d'H$^+$/mole d'Al$_2$O$_3$, de préférence de 0,01 à 1,5 mole d'H$^+$/mole d'Al$_2$O$_3$ et notamment de 0,05 à 1,0 mole d'H$^+$/mole d'Al$_2$O$_3$ soit obtenue.

**5.** Procédé selon l'une quelconque des revendications précédentes, dans lequel au moins 97 % en volume des particules de l'oxyhydrate d'aluminium ont une taille inférieure ou égale à 100 pm, en termes de diamètre moyen.

**6.** Procédé selon l'une quelconque des revendications 1 à 4, dans lequel l'oxyhydrate d'aluminium présente un spectre de tailles de particules selon lequel :

91 % en volume ≤ 90 $\mu$m,
51 % en volume ≤ 45 $\mu$m,
27 % en volume ≤ 25 $\mu$m,

à chaque fois en termes de diamètre de particule moyen.

**7.** Procédé selon l'une quelconque des revendications précédentes, dans lequel l'oxyhydrate d'aluminium est présent en une quantité de 10 % en poids à 40 % en poids d'oxyde d'aluminium, par rapport au poids total du produit final.

**8.** Procédé selon l'une quelconque des revendications précédentes, dans lequel la calcination a lieu à une température dans la plage allant de 500 à 700 °C pendant 6 heures ou moins, de préférence dans la plage allant de 550 °C à 600 °C pendant 1 à 5 heures.

**9.** Procédé selon l'une quelconque des revendications précédentes, dans lequel la zéolithe H peut être obtenue par un procédé comprenant les étapes suivantes :

(i) la fabrication de cristallites d'alumosilicate ayant un diamètre moyen allant de 0,01 $\mu$m ou plus à moins de 0,1 $\mu$m par mise en réaction d'une source de silicium, d'une source d'aluminium, d'une source d'alcali et d'une matrice à une température de réaction supérieure ou égale à 90 °C,
(ii) la séparation et éventuellement la calcination intermédiaire des cristallites de l'étape (i),
(iii) la mise en réaction des cristallites d'alumosilicate de l'étape (ii) avec un composé échangeur d'ions,
(iv) la séparation et éventuellement la calcination du produit obtenu à l'étape (iii).

**10.** Catalyseur, pouvant être obtenu par un procédé selon l'une quelconque des revendications 1 à 9.

**11.** Catalyseur selon la revendication 10, dont le volume poreux, déterminé par porosimétrie au mercure, est de 0,31 à 0,44 cm$^3$/g, de préférence de 0,38 à 0,44 cm$^3$/g et de manière particulièrement préférée de 0,41 à 0,44 cm$^3$/g.

**12.** Catalyseur selon la revendication 10 ou 11, dont la résistance à une pression latérale moyenne est de 7,8 N/mm ou plus, notamment de 7,8 à 14,7 N/mm.

**13.** Catalyseur selon l'une quelconque des revendications 10 à 12, dans lequel le catalyseur présente une conversion du méthanol moyenne dans un procédé de conversion de méthanol en oléfines, moyennée sur 200 à 400 heures dans le courant, de 96 % ou plus, notamment de 97 %.

**14.** Catalyseur selon l'une quelconque des revendications 10 à 13, dans lequel le catalyseur présente une sélectivité lors de la fabrication d'oléfines en C$_{2-4}$ dans un procédé de conversion de méthanol en oléfines, moyennée sur 200 à 400 heures dans le courant, de 65,0 % ou plus, de préférence de 68,3 % ou plus, notamment de 72,0 % ou plus.

**15.** Utilisation d'un catalyseur selon l'une quelconque des revendications 10 à 14 dans un procédé de conversion de méthanol en oléfines ou d'un catalyseur selon l'une quelconque des revendications 10 à 12 dans un procédé de conversion d'oléfines en oléfines.

**EP 2 593 219 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3702886 A
- DE 2822725 A
- DE 2405909 A
- FR 2217408 A
- US 4025572 A
- EP 0369364 A1

- EP 1892229 A1
- WO 2011061196 A1
- EP 1424128 A1
- DE 10027159 A1
- DE 10000889 A1
- EP 1424128 A